# EUROPÄISCHE PATENTSCHRIFT

(11) **EP 0 816 314 B1**
(45) Veröffentlichungstag und Bekanntmachung des Hinweises auf die Patenterteilung: **14.11.2001**
(21) Anmeldenummer: 97109996.5
(22) Anmeldetag: 19.06.1997
(51) Int. Cl.: C07C 17/38, C07C 17/386, C07C 17/383, C07C 19/03, C07C 41/42, C07C 43/04

(54) **Verfahren zur Trennung von Gemischen aus Dimethylether und Chlormethan**
Process for the separation of mixtures of dimethyl ether and chloromethane
Procédé pour la séparation de mélanges d'éther diméthylique et de chlorométhane

(30) Priorität: 25.06.1996 DE 19625284
(43) Veröffentlichungstag der Anmeldung: 07.01.1998
(73) Patentinhaber: HOECHST AKTIENGESELLSCHAFT, 65929 Frankfurt am Main (DE)
(72) Erfinder: Roth, Peter, Dl., 65817 Eppstein (DE); Leistner, Erhard, Dl., 35619 Braunfels (DE); Haverkamp, Hans, Dl., 65817 Eppstein (DE); Wendel, Wolfgang, Dr., 65779 Kelkheim (DE); Kleiber, Michael, Dr., 65929 Frankfurt (DE)
(74) Vertreter: Schweitzer, Klaus, Dr.

(56) Entgegenhaltungen:
- DD-A- 142 183
- US-A- 2 421 441
- US-A- 5 092 966
- DATABASE WPI Section Ch, Week 8036 Derwent Publications Ltd., London, GB; Class E16, AN 80-62426C XP002040756 & DD 142 183 A (BERGER K H) , 11.Juni 1980
- Klaus Sattler, "Thermische Trennverfahren, Grundlagen, Auslegung, Apparate", VCH Weinheim, 2.Aufl., Seiten 498-507

## Beschreibung

Die vorliegende Erfindung betrifft ein Verfahren zur Trennung von Gemischen aus Dimethylether (DME) und Chlormethan (CM).

Chlormethan besitzt technische Bedeutung als Ausgangsprodukt für die Herstellung von Fluorchlorkohlenwasserstoffen, die beispielsweise als Treibgase Verwendung finden. Dimethylether wird in zunehmendem Maße als Treibmittel in Spraydosen eingesetzt, da es halogenfrei ist, und somit ein geringes Ozonabbaupotential aufweist.

Die zu Dimethylether und Chlormethan führenden Reaktionen laufen unter sehr ähnlichen Bedingungen ab:

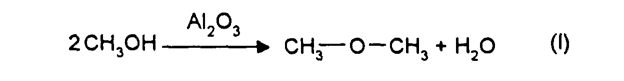

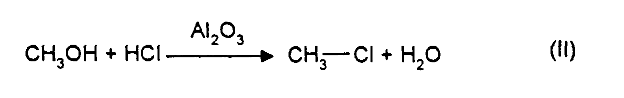

Bei der Chlormethanherstellung, die gemäß Formelzeile (II) beispielsweise an γ-Al₂O₃-Kontakten erfolgen kann, kommt es daher stets auch zur Bildung von Dimethylether gemäß Formelzeile (I). Das so entstandene Chlormethan/Dimethylether-Gemisch ist nach dem Stand der Technik destillativ nicht aufarbeitbar, da die Siedepunkte der Komponenten (Dimethylether: Kₚ =-24,9°C, Chlormethan: Kₚ = -23,7°C) sehr nahe beieinander liegen, und die Komponenten außerdem ein Azeotrop bilden.

Die Aufarbeitung des Gemisches aus Dimethylether und Chlormethan zur Gewinnung des reinen Chlormethans erfolgte bislang durch Hydrolyse des Dimethylethers mit Schwefelsäure, wodurch dieses wertvolle Nebenprodukt der Chlormethan-Herstellung verloren ging.

Die Aufgabe bestand somit darin, ein Trennverfahren für Chlormethan/Dimethylether-Gemische zu entwickeln, durch das beide Verbindungen in reiner Form isoliert werden können.

Diese Aufgabe wurde erfindungsgemäß durch einen zweistufigen Destillationsprozeß gelöst, in dem in einer Stufe ein Extraktionsmittel verwendet wird.

Gegenstand der Erfindung ist somit ein Verfahren zur Trennung von Chlormethan und Dimethylether durch Extraktivdestillation, dadurch gekennzeichnet, daß man
a) ein Dimethylether/Chlormethan-Gemisch in eine Extraktivdestillationskolonne einspeist,
b) im oberen Teil der Extraktivdestillationskolonne Wasser als Extraktionsmittel aufgibt,
c) am Extraktivdestillationskolonnenkopf Chlormethan als Kopfdampf abzieht,
d) vom Sumpf der Extraktivdestillationskolonne ein Gemisch aus Dimethylether und Extraktionsmittel abzieht,
e) das in Schritt d) abgezogene Gemisch in eine Destillationskolonne einspeist,
f) am Kopf der Destillationskolonne Dimethylether als Kopfdampf abzieht,
g) am Sumpf der Destillationskolonne das reine Extraktionsmittel abzieht und dann erneut der Extraktivdestillationskolonne zuführt.

DD-142 183 beschreibt ein Verfahren zur Trennung von Wasser, Methanol und den Chlormethanen Methylenchlorid, Chloroform und Tetrachlormethan durch Extraktivdestillation.

Die Destillationskolonnen können von einer beliebigen geeigneten Bauart sein, bevorzugt ist die Verwendung von Füllkörper- und Packungskolonnen. Vorzugsweise wird die Extraktivdestillation bei Drucken zwischen 1 und 25 bar durchgeführt.

In einer bevorzugten Ausführungsform der Erfindung wird das in Schritt e) genannte Gemisch vor der Einspeisung in die Destillationskolonne über einen Wärmetauscher geführt, in dem es erwärmt wird. Ebenfalls bevorzugt ist, das in Schritt g) genannte Extraktionsmittel über den genannten Wärmetauscher vorzukühlen, und es vor der Einspeisung in die Extraktivdestillationskolonne mit einem Kühler auf eine möglichst tiefe Temperatur abzukühlen.

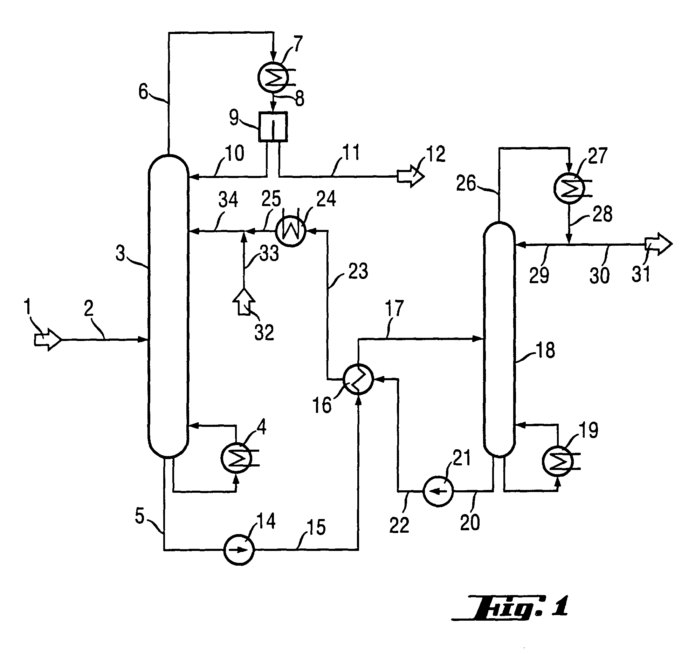

Fig. 1 zeigt ein Fließschema einer bevorzugten Ausführungsform des erfindungsgemäßen Verfahrens, das im folgenden näher erläutert wird. Ein Gemisch aus Chlormethan und Dimethylether (1) wird über Leitung (2) in eine Extraktivdestillationskolonne (3) eingespeist, in der die Trennung von Chlormethan und Dimethylether erfolgt. Im oberen Teil der Extraktivdestillationskolonne (3) wird über Leitung (34) das kalte Extraktionsmittel aufgegeben, dessen Temperatur vorzugsweise zwischen 5 und 50 °C beträgt. Als Extraktionsmittel dient Wasser. Die Aufgabe des Extraktionsmittels in die Extraktivdestillationskolonne (3) bewirkt, daß sich Dimethylether im Sumpf niederschlägt, und Chlormethan als Kopfdampf entnommen wird und über Leitung (6) in einen Kondensator (7) geleitet werden kann, wo es niedergeschlagen werden kann. Das Kondensat aus dem Kondensator (7) kann über Leitung (8) in einem Phasenabscheider (9) geleitet werden. Von dort gelangt die Chlormethan-Phase über die Leitung (11) zum Produktlager (12). Die zweit, wäßrige Phase kann vom Phasenabscheider (9) über Leitung (10) zur Extraktivdestillationskolonne (3) zurückgeführt werden, um wieder als Extraktionsmittel eingesetzt zu werden.

Im Sumpf der Extraktivdestillationskolonne (3), der mit dem Verdampfer (4) beheizt wird, sammelt sich das Gemisch aus Dimethylether und dem Extraktionsmittel an. Dieses Gemisch wird über Leitung (5) zur Pumpe (14), von dort über Leitung (15) gegebenenfalls zum Wärmetauscher (16), von dort über Leitung (17) zur Destillationskolonne (18) geführt. In der Destillationskolonne (18) wird Dimethylether vom Extraktionsmittel getrennt. Als Kopfdampf wird über Leitung (26) Dimethylether abgenommen, welches im Kondensator (27) niedergeschlagen werden kann. Das Kondensat kann teilweise über Leitung (28) und (29) in die Destillationskolonne (18) zurückgeführt werden, ansonsten wird es über Leitung (28) und (30) in das Produktlager (31) verbracht. Im Sumpf der Destillationskolonne (18), der über den Verdampfer (19) beheizt wird, fällt das Extraktionsmittel an. Dieses kann über Leitung (20), die Pumpe (21) und Leitung (22) zur Abkühlung dem Wärmetauscher (16) zugeführt werden, von wo es über Leitung (23) in einen Kühler (24) gelangen kann, um von dort über die Leitungen (25) und (34) wieder in die Extraktivdestillationskolonne (3) aufgegeben zu werden. Zur Ergänzung von Verlusten wird frisches Extraktionsmittel (32) über Leitung (33) zugeführt.

Das Verfahren kann in allen Teilen der Anlage sowohl unter Normaldruck als auch unter erhöhtem Druck durchgeführt werden. Die Fahrweise bei Drucken zwischen 1 und 25 bar ist bevorzugt, da sonst wegen der niedrigen Siedepunkte von Dimethylether und Chlormethan Kälte für die Kondensation der Kopfdämpfe erforderlich ist.

### Ausführungsbeispiel:

Ein Produktgemisch, bestehend aus 1,8 t/h Dimethylether und 7,3 t/h Chlormethan wird einer Extraktivdestillationskolonne (3) zugeführt. Diese Extraktivdestillationskolonne wird bei 10 bar betrieben, wodurch Chlormethan bei 40°C kondensiert werden kann. Extraktionsmittel ist Wasser, es werden 36 t/h bei 35°C in die Extraktivdestillationskolonne (3) eingespeist. Unter den herrschenden Bedingungen beträgt der Siedepunkt des Sumpfproduktes der Extraktivdestillationskolonne (3) 124°C. Der im Extraktionsmittel gelöste Anteil an Chlormethan wird im unteren Teil der Kolonne ausgestrippt. Das Sumpfprodukt besteht aus 1,8 t/h Dimethylether und 36 t/h Extraktionswasser, der Anteil an Chlormethan im Produktstrom beträgt 1 ppm. Als Kopfprodukt der Extraktivdestillation fallen 7,3 t/h Chlormethan an, der Dimethylether-Anteil beträgt hier weniger als 20 ppm. Der Sumpfablauf der Extraktivdestillationskolonne wird in die Destillationskolonne (18) eingespeist, wo die Trennung von Dimethylether und Extraktionswasser stattfindet. Hier fallen als Kopfprodukt 1,8 t/h Dimethylether mit einer Temperatur von etwa 40°C an. Der Chlormethan-Anteil liegt hier bei etwa 20 ppm, da das gesamte Chlormethan aus dem Sumpfablauf der Extraktivdestillationskolonne (3) in den Dimethylether übergeht. Das Sumpfprodukt der Destillationskolonne (18) besteht aus reinem Extraktionswasser von einer Temperatur von etwa 170°C und wird nach der Kühlung auf 35°C erneut in die Extraktivdestillationskolonne (3) eingespeist.

## Patentansprüche

1. Verfahren zur Trennung von Chlormethan und Dimethylether durch Extraktivdestillation, **dadurch gekennzeichnet, daß** man
a) ein Dimethylether/Chlormethan-Gemisch in eine Extraktivdestillationskolonne eingespeist,
b) im oberen Teil der Extraktivdestillationskolonne Wasser als Extraktionsmittel aufgibt,
c) am Extraktivdestillationskolonnenkopf Chlormethan als Kopfdampf abzieht,
d) vom Sumpf der Extraktivdestillationskolonne ein Gemisch aus Dimethylether und Extraktionsmittel abzieht,
e) das in Schritt d) abgezogene Gemisch in eine Destillationskolonne einspeist,
f) am Kopf der Destillationskolonne Dimethylether als Kopfdampf abzieht,
g) am Sumpf der Destillationskolonne das reine Extraktionsmittel abzieht und dann erneut der Extraktivdestillationskolonne zuführt.

2. Verfahren nach Anspruch 1, **dadurch gekennzeichnet, daß** die nach Schritt a) beschriebene Extraktivdestillation bei Drucken zwischen 1 und 25 bar durchgeführt wird.

3. Verfahren nach Anspruch 1 oder 2, **dadurch gekennzeichnet, daß** die in den Schritten c) und f) entnommenen Kopfprodukte vor der weiteren Verwendung kondensiert werden.

4. Verfahren nach einem der Ansprüche 1 bis 3, **dadurch gekennzeichnet, daß** alle verwendeten Kolonnen als Füllkörper- oder Packungskolonnen ausgeführt werden.

5. Verfahren nach einem der Ansprüche 1 bis 4, **dadurch gekennzeichnet, daß** das Extraktionsmittel Wasser in Schritt b) mit einer Temperatur zwischen 5 und 50 °C in die Extraktivdestillation eingespeist wird.

6. Verfahren nach einem der Ansprüche 1 bis 5, **dadurch gekennzeichnet, daß** in Schritt e) das dort genannte Gemisch vor der Einspeisung in die Destillationskolonne vorgewärmt wird, und daß das in Schritt g) genannte Extraktivmittel vor der Einspeisung in die Extraktivdestillationskolonne abgekühlt wird.

7. Verfahren nach Anspruch 6, **dadurch gekennzeichnet, daß** in Schritt e) das Gemisch über einen Wärmetauscher vorgewärmt wird, und daß in Schritt g) das Extraktivmittel über den Wärmetauscher vorgekühlt und über einen Kühler abgekühlt wird.

8. Verfahren nach einem der Ansprüche 1 bis 7, **dadurch gekennzeichnet, daß** man
1) das in Schritt c) entnommene Produkt kondensiert, das Kondensat in einem Phasenabscheider trennt, die Extraktionsmittelphase der Extraktivdestillationskolonne wieder zuführt, und das verbleibende Chlormethan entnimmt, und/oder
2) das in Schritt f) entnommene Produkt kondensiert und einen Teil des so entstandenen Kondensats teilweise dem Kopf der Destillationskolonne zuführt.

## Claims

1. A process for the separation of chloromethane and dimethyl ether by extractive distillation, which comprises
a) feeding a dimethyl ether/chloromethane mixture into an extractive distillation column,
b) adding water as extractant in the top part of the extractive distillation column,
c) taking off chloromethane vapor at the top of the extractive distillation column,
d) taking off a mixture of dimethyl ether and extractant at the bottom of the extractive distillation column,
e) feeding the mixture taken off in step d) into a distillation column,
f) taking off dimethyl ether vapor at the top of the distillation column,
g) taking off the pure extractant at the bottom of the distillation column and then feeding it again into the extractive distillation column.

2. The process as claimed in claim 1, wherein the extractive distillation described after step a) is carried out under pressures between 1 and 25 bar.

3. The process as claimed in claim 1 or 2, wherein the top products taken off in steps c) and f) are condensed before being used further.

4. The process as claimed in any of claims 1 to 3, wherein all the columns used are designed as packed columns.

5. The process as claimed in any of claims 1 to 4, wherein the extractant water in step b) is fed at a temperature between 5 and 50°C into the extractive distillation.

6. The process as claimed in any of claims 1 to 5, wherein the mixture mentioned in step e) is preheated before being fed into the distillation column, and wherein the extractant mentioned in step g) is cooled before being fed into the extractive distillation column.

7. The process as claimed in claim 6, wherein the mixture in step e) is preheated in a heat exchanger, and wherein the extractant in step g) is precooled through the heat exchanger and cooled through a cooler.

8. The process as claimed in any of claims 1 to 7, wherein
1) the product taken off in step c) is condensed, the condensate is separated in a phase separator, the extractant phase is returned to the extractive distillation column, and the remaining chloromethane is taken off,
and/or
2) the product taken off in step f) is condensed, and part of the condensate produced in this way is fed in part to the top of the distillation column.

## Revendications

1. Procédé pour la séparation de chlorométhane et d'éther diméthylique par distillation extractive, **caractérisé en ce que**
a) on introduit un mélange d'éther diméthylique/chlorométhane dans une colonne de distillation extractive,
b) dans la partie supérieure de la colonne de distillation extractive on introduit de l'eau en tant qu'agent d'extraction,
c) on évacue du chlorométhane en tant que vapeur de tête à la tête de la colonne de distillation extractive,
d) on soutire du pied de la colonne de distillation extractive un mélange constitué d'éther diméthylique et d'agent d'extraction,
e) on introduit dans une colonne de distillation le mélange soutiré dans l'étape d),
f) on évacue l'éther diméthylique sous forme de vapeur de tête à la tête de la colonne de distillation,
g) on soutire au pied de la colonne de distillation l'agent d'extraction pur, et on l'envoie ensuite de nouveau à la colonne de distillation extractive.

2. Procédé selon la revendication 1, **caractérisé en ce que** la distillation extractive décrite selon l'étape a) est effectuée sous des pressions comprises entre 1 et 25 bars.

3. Procédé selon la revendication 1 ou 2, **caractérisé en ce que** les produits de tête prélevés dans les étapes c) et f) sont condensés avant leur nouvelle utilisation.

4. Procédé selon l'une quelconque des revendications 1 à 3, **caractérisé en ce que** toutes les colonnes utilisées sont réalisées sous forme de colonne à garnissage ou corps de remplissage.

5. Procédé selon l'une quelconque des revendications 1 à 4, **caractérisé en ce que** l'agent d'extraction eau dans l'étape b) est introduit à une température comprise entre 5 et 50°C dans la distillation extractive.

6. Procédé selon l'une quelconque des revendications 1 à 5, **caractérisé en ce que** dans l'étape e) le mélange qui y est cité est préchauffé avant l'introduction dans la colonne de distillation, et **en ce que** l'agent d'extraction mentionné dans l'étape g) est refroidi avant l'introduction dans la colonne de distillation extractive.

7. Procédé selon la revendication 6, **caractérisé en ce que**, dans l'étape e), le mélange est préchauffé sur un échangeur thermique et **en ce que**, dans l'étape g), l'agent d'extraction est pré-refroidi sur l'échangeur thermique, puis refroidi sur un refroidisseur.

8. Procédé selon l'une quelconque des revendications 1 à 7, **caractérisé en ce que**
1) on condense le produit prélevé dans l'étape c), on sépare le condensat dans un séparateur de phases, on renvoie la phase contenant l'agent d'extraction à la colonne de distillation extractive, et on prélève le chlorométhane restant, et/ou
2) on condense le produit prélevé dans l'étape f )et une partie du condensat ainsi obtenu est envoyé en partie à la tête de la colonne de distillation.
